# EUROPEAN PATENT APPLICATION

(11) **EP 4 534 094 A1**
(43) Date of publication of application: **09.04.2025**
(21) Application number: 23812038.0
(22) Date of filing: 10.05.2023
(51) Int. Cl.: A61K 35/747, A61P 3/00, A61P 1/16, A61P 9/00, A23L 33/135, C12N 1/20

(54) **COMPOSITION FOR PREVENTING, TREATING, OR IMPROVING METABOLIC DISEASES COMPRISING LACTOBACILLUS PLANTARUM NCHBL-004 STRAIN OR CULTURE MEDIUM THEREOF**

(30) Priority: 25.05.2022 KR 20220064168; 06.10.2022 KR 20220128076
(71) Applicant: Industry Foundation of Chonnam National University, Gwangju 61186 (KR)
(72) Inventor: PARK, Jong Hwan, Gwangju 61746 (KR); KOH, Hong Bum, Gwangju 61149 (KR); HUH, Joo Young, Gwangju 61424 (KR); JUNG, Do Hyeon, Gwangju 61684 (KR); SONG, Eun Jung, Gwangju 61220 (KR); MA, Eun Bi, Gwangju 61982 (KR)
(74) Representative: Plougmann Vingtoft a/s
(86) International application number: PCT/KR2023/006344
(87) International publication number: WO 2023/229263

(57) **Abstract**

The present invention relates to a composition for preventing, treating, or improving metabolic diseases comprising *Lactobacillus plantarum* NCHBL-004 strain or a culture medium thereof, and the composition not only inhibits adipocyte differentiation and inhibits weight gain, but also exhibits a blood sugar lowering effect, and can thus be effectively used for preventing, treating, or improving metabolic diseases.

## Description

### Technical Field

The present disclosure was carried out with support from the Ministry of Education, under Project ID. Number 1320213639 and Sub-project Number 2021-3639. The National Research Foundation of Korea served as the research management agency, with the project titled "Support Project for Practicalization of Korean Creative Assets" and the research project titled "Evaluation of the Efficacy of Honeybee-Derived Lactic Acid Bacteria in Reducing Body Fat and Lowering Blood Glucose Levels in Animal Models." The principal institution overseeing the project is the Chonnam National University Industry-Academic Cooperation Foundation, and the research period spans from October 1, 2021, to January 31, 2022.

This patent application claims the benefit of and priority to Korean Patent Application No. 10-2022-0064168, filed on May 25, 2022, with the Korean Intellectual Property Office, and Korean Patent Application No. 10-2022-0128076, filed on October 6, 2022, with the Korean Intellectual Property Office, the disclosures of which are incorporated herein by reference.

The present disclosure relates to a composition including *Lactobacillus plantarum* NCHBL-004 strain or a culture thereof for the prevention, treatment, or alleviation of metabolic diseases. More specifically, the present disclosure relates to a technology for applying either a culture or live bacteria of *Lactobacillus plantarum* NCHBL-004 to the prevention, treatment, or alleviation of metabolic diseases, including obesity, diabetes, and non-alcoholic fatty liver disease (NAFLD).

### Background Art

Recently, the prevalence of adult diseases such as obesity and diabetes has been on the rise, driven by Westernized dietary habits, genetic predisposition, and environmental factors. According to the Korean Society for the Study of Obesity, the rate of severe obesity in Korea increased by approximately 72% over a decade, from 3.5% in 2009 to 6.01% in 2018. If this trend continues, it is projected that by 2030, one in ten individuals in Korea will be classified as severely obese.

The associated social costs cannot be ignored. In 2008 alone, Korea spent approximately 1.7923 trillion KRW on obesity treatment, with these costs rising annually. While research is being conducted to identify substances that can help prevent and treat obesity, there is a growing interest in the development of functional health foods. Obesity is clearly classified as a disease that not only represents an excessive accumulation of fat but can also lead to other severe health conditions that threaten life. Obesity can lead to comorbidities such as osteoarthritis and sleep apnea, as well as metabolic complications such as non-alcoholic fatty liver disease (NAFLD), diabetes, hypertension, hyperlipidemia, severe cardiovascular diseases, and even cancer.

Non-alcoholic fatty liver disease (NAFLD) accounts for 70-90% of chronic hepatitis cases. When oxidative stress or insulin resistance increases, inflammatory responses can occur in the liver, which may ultimately progress to severe fatty liver disease or non-alcoholic steatohepatitis (NASH). Once the disease advances to steatohepatitis, it poses a heightened risk of developing into cirrhosis, liver failure, or hepatocellular carcinoma, which can lead to mortality. Furthermore, it may cause other chronic diseases, such as cardiovascular disease, underscoring the importance of breaking these links.

Although many pharmaceutical companies worldwide are engaged in the development of NASH treatments, no product has yet been approved by regulatory authorities. Current obesity medications primarily work by inducing satiety through direct action on the brain, thereby suppressing appetite. Obesity drugs like orlistat, which reduces fat absorption, facilitate weight loss by decreasing fat intake. Satiety-inducing anti-obesity drugs are classified and regulated as psychotropic substances due to dependency and tolerance issues, and they often increase the concentration of neurotransmitters like norepinephrine or serotonin at synapses, thereby inducing satiety. These drugs may also trigger satiety by stimulating serotonin or adrenergic receptors.

However, the prolonged use of these drugs for over three months can lead to side effects such as fatigue, depression, hallucinations, and sleep disorders, necessitating caution regarding drug addiction risks. Therefore, in the treatment of metabolic syndrome caused by obesity, there is an emerging demand for therapeutic strategies with novel mechanisms that offer effective anti-obesity and metabolic disease-improvement effects with minimal side effects.

*Lactobacillus* species are lactic acid bacteria that undergo homo- or heterofermentation. Commonly found in the fermentation processes of dairy products and vegetables, they are generally classified as beneficial bacteria. Recent studies have reported that administering beneficial intestinal bacteria such as *Lactobacillus* or *Bifidobacterium* (i.e., probiotics) has demonstrated effects on weight reduction, alleviation of fatty liver and inflammation in adipose tissues, and correction of gut microbiota imbalance in obesity models induced by high-fat diets.

### Disclosure of Invention

### Technical Problem

Research conducted by the present inventors have confirmed that *Lactobacillus plantarum* derived from honeybees demonstrates a strong inhibitory effect on adipocyte differentiation and an excellent effect on obesity alleviation.

Accordingly, the present disclosure aims to provide a pharmaceutical composition including *Lactobacillus plantarum* NCHBL-004 strain or a culture thereof for the prevention or treatment of metabolic diseases.

Also, the present disclosure is to provide a health-functional food composition including *Lactobacillus plantarum* NCHBL-004 strain or a culture thereof for the alleviation of metabolic diseases.

Furthermore, the present disclosure relates to a use of *Lactobacillus plantarum* NCHBL-004 strain or a culture thereof for the prevention, treatment, or alleviation of metabolic diseases.

### Solution to Problem

The present disclosure relates to a composition including *Lactobacillus plantarum* NCHBL-004 strain or a culture thereof for the prevention, treatment, or alleviation of metabolic diseases. The composition according to the present disclosure not only inhibits adipocyte differentiation and weight gain but also exhibits blood glucose-lowering effects.

Research conducted by the present inventors found that the use of live bacteria or a culture of *Lactobacillus plantarum* derived from honeybees inhibits adipocyte differentiation from preadipocytes and demonstrates weight gain inhibition and blood glucose-lowering effects in an obesity-induced mouse model following a high-fat diet.

Below, a detailed description will be given of the present disclosure.

One aspect of the present disclosure is a pharmaceutical composition including *Lactobacillus plantarum* NCHBL-004 strain or a culture thereof for the prevention or treatment of metabolic diseases.

In the present disclosure, the strain may be deposited under accession number KCTC14909BP.

The strain may be in the form of live bacteria, dead bacteria, or a mixture thereof, and, for example, may be live bacteria, but is not limited thereto.

In the present disclosure, the composition may contain the strain at a concentration of 5x 10⁵ to 5x10¹¹ CFU/ml, preferably at 5x10⁵ to 5x10⁹ CFU/ml, 5x10⁵ to 5x10⁷ CFU/ml, or 5x10⁷ to 5x10¹¹ CFU/ml, and, for example, may be at a concentration of 5x10⁷ to 5x10⁹ CFU/ml, but is not limited thereto.

The culture may be the culture supernatant obtained by culturing the strain and removing the bacterial body mass, a concentrate, fraction, or lyophilizate thereof, and, for example, may be the culture supernatant, but is not limited thereto.

The metabolic disease may be at least one selected from the group consisting of obesity, diabetes, non-alcoholic fatty liver disease (NAFLD), non-alcoholic steatohepatitis (NASH), hypertension, hyperlipidemia, cardiovascular disease, and hyperinsulinemia.

The pharmaceutical composition may include *Lactobacillus plantarum* NCHBL-004 strain or a culture thereof in a pharmaceutically effective amount and/or a pharmaceutically acceptable carrier.

The term "pharmaceutically effective amount", as used herein, refers to an amount sufficient to achieve the efficacy or activity of the culture of *Lactobacillus sakei* CVL-001 strain described above.

The pharmaceutically acceptable carrier included in the pharmaceutical composition is conventionally used in formulation, and may include lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methylcellulose, methylparaben, propylparaben, talc, magnesium stearate, and mineral oil, but is not limited thereto. The pharmaceutical composition of the present disclosure may further include lubricants, wetting agents, sweeteners, flavoring agents, emulsifiers, suspending agents, and preservatives in addition to the ingredients mentioned above.

The pharmaceutical composition according to the present disclosure may be administered to mammals, including humans, by various routes. All administration modes that are used commonly may be contemplated, and for example, administration may take oral, dermal, intravenous, intramuscular, or subcutaneous routes, with preference for an oral route.

An appropriate dosage of the pharmaceutical composition may vary depending on factors such as the formulation method, administration method, age, body weight, sex, pathological condition of the patient, food intake, administration time, administration route, excretion rate, and response sensitivity. A skilled practitioner can readily determine and prescribe a dosage effective for the desired treatment or prevention.

The pharmaceutical composition of the present disclosure may be formulated, along with a pharmaceutically acceptable carrier and/or excipient, in a unit dosage form or incorporated into a multi-dose container using methods well-known to those skilled in the art. The formulation may be in the form of a solution, suspension, or emulsion in an oil or aqueous medium, an extract, pulvis, granules, tablets, capsules, or gels (e.g., hydrogel) and may further include dispersing or stabilizing agents.

Another aspect of the present disclosure is a health-functional food composition containing *Lactobacillus plantarum* NCHBL-004 strain or a culture thereof for the alleviation of metabolic diseases.

In the present disclosure, the strain may be deposited under accession number KCTC14909BP.

The strain may be in the form of live bacteria, dead bacteria, or a mixture thereof, and, for example, may be live bacteria, but is not limited thereto.

The composition may contain the strain at a concentration of 5x10⁵ to 5x10¹¹ CFU/ml, preferably at 5×10⁵ to 5x10⁹ CFU/ml, 5x10⁵ to 5x10⁷ CFU/ml, or 5x10⁷ to 5x10¹¹ CFU/ml, and, for example, at a concentration of 5x10⁷ to 5x10⁹ CFU/ml, but is not limited thereto.

The culture may be a culture supernatant obtained by culturing the strain and removing the bacterial body mass, or a concentrate, fraction, or lyophilizate thereof, and, for example, may be a culture supernatant, but is not limited thereto.

The metabolic disease may be at least one selected from the group consisting of obesity, diabetes, non-alcoholic fatty liver disease, non-alcoholic steatohepatitis, hypertension, hyperlipidemia, cardiovascular disease, and hyperinsulinemia.

When using the health-functional food composition of the present disclosure as a food additive, it may be added directly or used with other foods or food ingredients, and it may be appropriately used following conventional methods. Typically, in the preparation of foods or beverages, the food composition of the present disclosure may be added in an amount of 15% by weight or less and preferably in an amount of 10% by weight or less, based on the weight of the raw material.

No particular limitations are imparted to the types of foods. Examples of foods to which the substance may be added include meat, sausages, bread, chocolate, candies, snacks, cookies, pizza, ramen, noodles, gum, dairy products including ice cream, various soups, beverages, tea, drinks, alcoholic beverages, and vitamin complexes, encompassing all foods in the usual sense.

The beverage may contain various flavoring agents or natural carbohydrates as additional components. Examples of natural carbohydrates include monosaccharides such as glucose and fructose, disaccharides such as maltose and sucrose, and natural sweeteners like dextrin and cyclodextrin, as well as artificial sweeteners such as saccharin and aspartame. The ratio of the natural carbohydrate can be appropriately determined by those skilled in the art.

In addition, the health-functional food composition of the present disclosure may contain various nutrients, vitamins, electrolytes, flavoring agents, colorants, pectic acid and salts thereof, alginic acid and salts thereof, organic acids, protective colloidal thickeners, pH adjusters, stabilizers, preservatives, glycerin, alcohol, and carbonating agents used in carbonated beverages. Moreover, the health-functional food composition of the present disclosure may contain pulp for the preparation of natural fruit juices, fruit juice drinks, and vegetable drinks. These ingredients may be used independently or in combination. The ratio of such additives may also be appropriately selected by those skilled in the art.

### Advantageous Effects of Invention

The present disclosure relates to a composition including *Lactobacillus plantarum* NCHBL-004 strain or a culture thereof for the prevention, treatment, or alleviation of metabolic diseases. This composition not only inhibits adipocyte differentiation and weight gain but also demonstrates blood glucose-lowering effects, finding effective applications in the prevention, treatment, or alleviation of metabolic diseases.

### Brief Description of Drawings

FIG. 1 is a graph showing the cytotoxicity of culture supernatants of *Lactobacillus kunkeei* NCHBL-003 and *Lactobacillus plantarum* NCHBL-004 on 3T3-L1 preadipocytes according to an embodiment of the present disclosure.
FIG. 2a is a graph showing the inhibitory effect of culture supernatants of *Lactobacillus kunkeei* NCHBL-003 and *Lactobacillus plantarum* NCHBL-004 on adipocyte differentiation in preadipocytes, according to an embodiment of the present disclosure.
FIG. 2b shows photographic images illustrating the inhibitory effect of culture supernatants of *Lactobacillus kunkeei* NCHBL-003 and *Lactobacillus plantarum* NCHBL-004 on adipocyte differentiation in preadipocytes, according to an embodiment of the present disclosure.
FIG. 3 shows photographic images of the expression levels of transcription factors related to adipocyte differentiation, influenced by culture supernatants of *Lactobacillus kunkeei* NCHBL-003 and *Lactobacillus plantarum* NCHBL-004 in preadipocytes, according to an embodiment of the present disclosure.
FIG. 4a is a plot showing the weight gain suppression effect of live *Lactobacillus kunkeei* NCHBL-003 in an obesity-induced mouse model according to an embodiment of the present disclosure.
FIG. 4b is a plot showing the weight gain suppression effect of live *Lactobacillus plantarum* NCHBL-004 in an obesity-induced mouse model according to an embodiment of the present disclosure.
FIG. 5a is a plot showing the blood glucose-lowering effect in a glucose tolerance test for live *Lactobacillus kunkeei* NCHBL-003 in an obesity-induced mouse model, according to an embodiment of the present disclosure.
FIG. 5b is a plot showing the blood glucose-lowering effect of live *Lactobacillus plantarum* NCHBL-004 in an obesity-induced mouse model, as measured by a glucose tolerance test, according to an embodiment of the present disclosure.
FIG. 5c is a graph showing the area under the blood glucose-lowering curve in a glucose tolerance test for live *Lactobacillus kunkeei* NCHBL-003 in an obesity-induced mouse model, according to an embodiment of the present disclosure.
FIG. 5d is a graph showing the area under the blood glucose-lowering curve in a glucose tolerance test for live *Lactobacillus plantarum* NCHBL-004 in an obesity-induced mouse model, according to an embodiment of the present disclosure.
FIG. 6a is a plot showing the blood glucose-lowering effect of live *Lactobacillus kunkeei* NCHBL-003 in an obesity-induced mouse model, as measure by an insulin tolerance test, according to an embodiment of the present disclosure.
FIG. 6b is a plot showing the blood glucose-lowering effect of live *Lactobacillus plantarum* NCHBL-004 in an obesity-induced mouse model, as measured by an insulin tolerance test, according to an embodiment of the present disclosure.
FIG. 6c is a graph showing the area under the blood glucose-lowering curve in an insulin tolerance test for live *Lactobacillus kunkeei* NCHBL-003 in an obesity-induced mouse model, according to an embodiment of the present disclosure.
FIG. 6d is a graph showing the area under the blood glucose-lowering curve in an insulin tolerance test for live *Lactobacillus plantarum* NCHBL-004 in an obesity-induced mouse model, according to an embodiment of the present disclosure.
FIG. 7a is a graph showing the liver weight reduction effect in an obesity-induced mouse model for live *Lactobacillus kunkeei* NCHBL-003 and *Lactobacillus plantarum* NCHBL-004 according to an embodiment of the present disclosure.
FIG. 7b is a graph showing the subcutaneous fat weight reduction effect in an obesity-induced mouse model for live *Lactobacillus kunkeei* NCHBL-003 and *Lactobacillus plantarum* NCHBL-004 according to an embodiment of the present disclosure.
FIG. 8a shows photographic images of histological clinical outcomes of non-alcoholic fatty liver disease (NAFLD) in an obesity-induced mouse model, treated with live *Lactobacillus kunkeei* NCHBL-003 and live *Lactobacillus plantarum* NCHBL-004 according to an embodiment of the present disclosure.
FIG. 8b is a graph showing histological clinical outcomes of non-alcoholic fatty liver disease (NAFLD) in an obesity-induced mouse model, treated with live *Lactobacillus kunkeei* NCHBL-003 and live *Lactobacillus plantarum* NCHBL-004 according to an embodiment of the present disclosure.
FIG. 9a shows photographic images showing the adipocyte size in adipose tissue in an obesity-induced mouse model treated with live *Lactobacillus kunkeei* NCHBL-003 and live *Lactobacillus plantarum* NCHBL-004 according to an embodiment of the present disclosure.
FIG. 9b is a graph showing the adipocyte size in adipose tissue in an obesity-induced mouse model treated with live *Lactobacillus kunkeei* NCHBL-003 and live *Lactobacillus plantarum* NCHBL-004 according to an embodiment of the present disclosure.
FIG. 10 shows graphs of the expression levels of metabolism-related genes in adipose tissue in an obesity-induced mouse model treated with live *Lactobacillus kunkeei* NCHBL-003 according to an embodiment of the present disclosure.
FIG. 11 shows graphs of the expression levels of adipocyte differentiation-related genes in adipose tissue in an obesity-induced mouse model treated with live *Lactobacillus plantarum* NCHBL-004 according to an embodiment of the present disclosure.
FIG. 12 shows graphs of the expression levels of inflammation-related genes in adipose tissue in an obesity-induced mouse model treated with live *Lactobacillus plantarum* NCHBL-004 according to an embodiment of the present disclosure.

### Best Mode for Carrying out the Invention

The present disclosure pertains to a pharmaceutical composition including *Lactobacillus plantarum* NCHBL-004 strain or a culture thereof for the prevention or treatment of metabolic diseases.

### Mode for Carrying out the Invention

Hereinafter, a better understanding of the present disclosure may be obtained in the following examples, which are set forth to illustrate, but are not to be construed to limit, the present disclosure.

Throughout this specification, unless otherwise stated, the "%" used to indicate the concentration of a particular substance represents (weight/weight)% for solid/solid mixtures, (weight/volume)% for solid/liquid mixtures, and (volume/volume)% for liquid/liquid mixtures.

### EXAMPLE 1: Preparation of Culture Fluids of Lactobacillus kunkeei NCHBL-003 and Lactobacillus plantarum NCHBL-004 Strains Isolated from Honeybees

### 1-1. Isolation of Lactic Acid Bacteria

To isolate lactic acid bacteria from the gut microbiota of honeybees, the target insects were first collected in early June from the Gwangju region and stored in sterilized collection containers under refrigeration for 10 minutes per individual. The gastrointestinal tracts were then aseptically extracted using microdissection tools in a clean bench. The gut microbiota of the honeybees was obtained by suspending the extracted gastrointestinal contents in 10 ml of a sterilized peptone physiological saline solution (0.9% w/v NaCl, 0.1% w/v Tween 80, 0.1% w/v peptone).

The lactic acid bacteria were isolated by serially diluting 500 µl of the suspension of gut homogenates in a liquid medium (Difco^{™} Lactobacilli MRS Broth, BD, USA) and streaking onto a solid medium (BBL^{™} LBS Agar, BD, USA) using a platinum loop. The bacteria were cultured with the addition of BD GasPak^{™} EZdp at 37°C until bacterial colonies were observed. Colonies on the LBS solid medium were identified as *Lactobacillus,* as assayed by Gram staining (Gram-positive) and a catalase-negative reaction upon mixing with 3% hydrogen peroxide. The isolated outdoor strains of *Lactobacillus* were preserved in MRS medium containing 15% glycerol and stored at -20°C for subsequent experiments.

Among the isolated strains, *Lactobacillus kunkeei* NCHBL-003 and *Lactobacillus plantarum* NCHBL-004 were phylogenetically identified by sequencing the 16S rRNA gene. Using the PHYDIT program, the 16S rRNA gene were compared with those of other *Lactobacillus* strains on the basis of the similarity of primary and secondary structures.

**TABLE 1**

| SEQ ID NO: | Name | Sequence |
|---|---|---|
| 1 | Lactobac illus plantaru m NCHBL-004 16S rRNA | TGCAGTCGAACGAACTCTGGTATTGATTGGTGCTTGCATCATGAT TTACATTTGAGTGAGTGGCGAACTGGTGAGTAACACGTGGGAAAC CTGCCCAGAAGCGGGGGATAACACCTGGAAACAGATGCTAATACC GCATAACAACTTGGACCGCATGGTCCGAGTTTGAAAGATGGCTTC GGCTATCACTTTTGGATGGTCCCGCGGCGTATTAGCTAGATGGTG GGGTAACGGCTCACCATGGCAATGATACGTAGCCGACCTGAGAGG GTAATCGGCCACATTGGGACTGAGACACGGCCCAAACTCCTACGG GAGGCAGCAGTAGGGAATCTTCCACAATGGACGAAAGTCTGATGG AGCAACGCCGCGTGAGTGAAGAAGGGTTTCGGCTCGTAAAACTCT GTTGTTAAAGAAGAACATATCTGAGAGTAACTGTTCAGGTATTGA CGGTATTTAACCAGAAAGCCACGGCTAACTACGTGCCAGCAGCCG CGGTAATACGTAGGTGGCAAGCGTTGTCCGGATTTATTGGGCGTA AAGCGAGCGCAGGCGGTTTTTTAAGTCTGATGTGAAAGCCTTCGG CTCAACCGAAGAAGTGCATCGGAAACTGGGAAACTTGAGTGCAGA AGAGGACAGTGGAACTCCATGTGTAGCGGTGAAATGCGTAGATAT ATGGAAGAACACCAGTGGCGAAGGCGGCTGTCTGGTCTGTAACTG ACGCTGAGGCTCGAAAGTATGGGTAGCAAACAGGATTAGATACCC TGGTAGTCCATACCGTAAACGATGAATGCTAAGTGTTGGAGGGTT TCCGCCCTTCAGTGCTGCAGCTAACGCATTAAGCATTCCGCCTGG GGAGTACGGCCGCAAGGCTGAAACTCAAAGGAATTGACGGGGGCC CGCACAAGCGGTGGAGCATGTGGTTTAATTCGAAGCTACGCGAAG AACCTTACCAGGTCTTGACATACTATGCAAATCTAAG |

### 1-2. Preparation of Culture of Strains

Live *Lactobacillus kunkeei* NCHBL-003 and *Lactobacillus plantarum* NCHBL-004 strains (1×10⁸ CFU/ml) isolated from the honeybee gut were cultured for 24 hours in DMEM (high glucose). After centrifugation, the supernatant was collected, adjusted to pH 7.4, and sterilized using a syringe filter (0.2 µm) before storage.

### EXAMPLE 2: Experiment for Inhibitory Effect on Adipocyte Differentiation (In Vitro)

### 2-1. Evaluation of Cytotoxicity

Cytotoxicity can be assessed by measuring lactate dehydrogenase (LDH) released from dead cells. 3T3-L1 preadipocyte cells purchased from the Korean Cell Line Bank were maintained by passages in DMEM supplemented with 10% bovine calf serum (BCS) and 1% penicillin-streptomycin (PS). Cells were seeded at a density of 1×10⁵ cells/ml in 0.2 ml of medium supplemented with 10% fetal bovine serum (FBS) and 1% PS in 48-well plates. Upon reaching 100% confluence (designated as Day -2) after inoculation on the plate, cells were further cultured for two additional days such that all cells cultured in the plates were in cell cycle arrest at the G1 phase.

To measure spontaneous LDH release, control wells were prepared without any treatment. Specifically, starting from Day 0, adipocyte differentiation of 3T3-L1 preadipocytes was induced by adding MDI (IBMX, dexamethasone, and insulin) to the medium containing 10% FBS. On Day 2, the medium was replaced with one containing only insulin, and from Day 4 onward, the medium was replaced every two days until the cells reached the differentiated state (Day 8).

To measure maximum LDH release, treated wells were prepared with the culture. Cells were prepared similarly to the control group, and on Day 0 (two days after reaching 100% confluence), the culture was added at concentrations of 12.5%, 25%, and 50%. The culture was diluted using DMEM medium.

After culturing the cells for 24 hours, an LDH assay was conducted to evaluate cytotoxicity. Wells with Triton X-100 solution were prepared, and after adding the LDH substrate mixture, absorbance was read to calculate the toxicity percentage.

Cytotoxicity was evaluated by calculating the percentage of LDH released from *Lactobacillus kunkeei* NCHBL-003 and *Lactobacillus plantarum* NCHBL-004 cultures (0%, 12.5%, 25%, 50%).

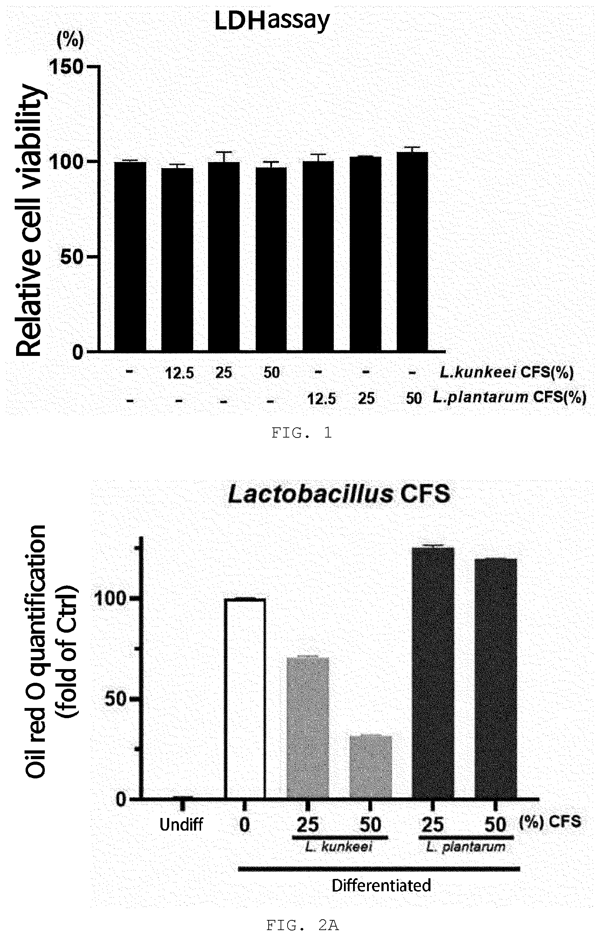

As shown in FIG. 1, no cytotoxicity was observed at any of the concentrations of culture treated on 3T3-L1 cells.

### 2-2. Evaluation of Inhibition on Adipocyte Differentiation

3T3-L1 preadipocyte cells were seeded at a density of 1×10⁵ cells/ml in 1 ml of medium containing 10% FBS and 1% PS in 12-well plates. On Day 0, two days after reaching 100% confluence, the culture (0%, 12.5%, 25%, 50%) and the differentiation inducer MDI were added to the cells.

On Day 2, the medium was replaced while the cells were treated with the culture (0%, 12.5%, 25%, 50%) and insulin. On Day 4, the medium was replaced again, and the culture (0%, 12.5%, 25%, 50%) were added. This process was repeated every two days until differentiation was complete. Once adipocyte differentiation was observed, the medium was replaced with 4% formalin for lipid staining. The cells were fixed in formalin for 20 minutes and washed twice with distilled water.

The cells were then stained for 20 minutes with Oil Red O staining solution, prepared by mixing Oil Red O dye and distilled water at a 6:4 ratio. After washing twice with distilled water, the cells were observed and photographed under a microscope. The Oil Red O stain was then extracted with 100% isopropanol, and the absorbance was read at 510 nm to quantitatively analyze the degree of differentiation.

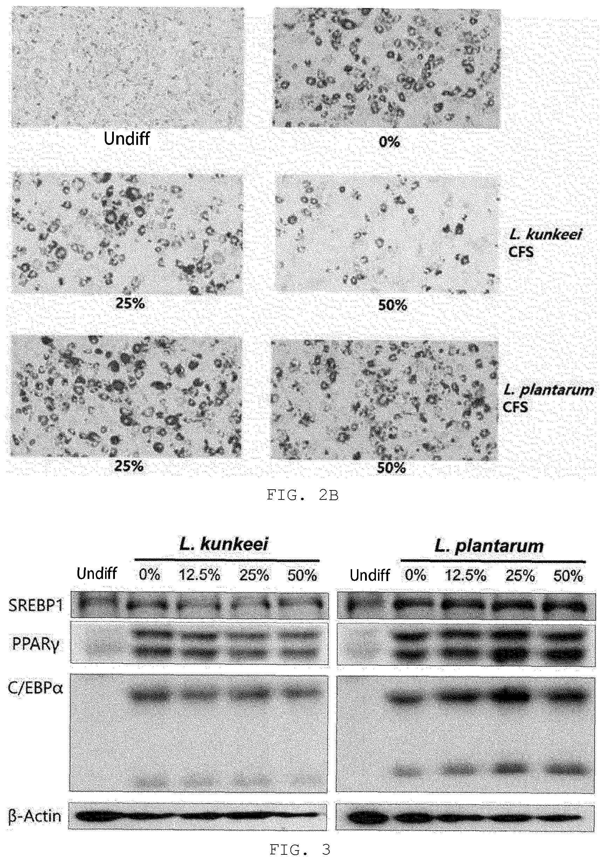

As shown in FIGS. 2a and 2b, treatment with MDI increased differentiation of 3T3-L1 cells into adipocytes, while treatment with *Lactobacillus kunkeei* NCHBL-003 culture significantly reduced the number of adipocytes in a dose-dependent manner. However, the number of adipocytes did not decrease with treatment using *Lactobacillus plantarum* NCHBL-004 culture.

From the data, it is understood that the culture fluid of *Lactobacillus kunkeei* NCHBL-003 inhibits differentiation into adipocytes.

### 2-3. Evaluation for Inhibition of Transcription Factors Related to Adipocyte Differentiation

During adipogenesis in adipose tissue, key transcription factors such as SREBP1c (sterol regulatory element-binding protein 1c), PPARγ (peroxisome proliferator-activated receptor-γ), and C/EBPα (CCAAT-enhancer-binding protein α) are involved in the processes of lipid biosynthesis, lipid droplet formation, and lipid accumulation. To assess the effect of the lactic acid bacteria culture on the expression of these transcription factors in 3T3-L1 cells differentiated into adipocytes, protein expression levels of these transcription factors were measured by Western blotting.

Specifically, 3T3-L1 cells were seeded at a density of 1×10⁵ cells/ml in 12-well plates and cultured as described previously with or without treatment of lactic acid bacteria culture until Day 8. The cells from each group were collected using a cell scraper, and proteins were extracted using a protein lysis buffer containing a protease inhibitor.

Equal amounts of protein were loaded onto an SDS-PAGE gel and separated by molecular weight, followed by transfer to a PVDF (Polyvinylidene fluoride) membrane. The transferred membrane was incubated with primary antibodies-SREBP1c (#ab28481, Abcam), PPARγ (#2443S, Cell Signaling Technology), C/EBPα (#8178T, Cell Signaling Technology), and β-actin (#sc-47778, Santa Cruz Biotechnology)-at 4°C for 18 hours, followed by washing with TBST buffer.

The membrane was then incubated with secondary antibodies-Anti-Rabbit IgG, Anti-Mouse IgG, and HRP-linked Secondary Antibody (#31640, Invitrogen)-at room temperature for 2 hours and washed with TBST. A detection reagent was applied to the membrane, and the protein expression levels of the target proteins were visualized and quantified.

As shown in FIG. 3, the protein expression levels of the transcription factors increased with MDI-induced adipocyte differentiation, while treatment with *Lactobacillus kunkeei* NCHBL-003 culture fluid significantly reduced the expression levels of these transcription factors in a concentration-dependent manner. However, *Lactobacillus plantarum* NCHBL-004 culture fluid treatment did not reduce the expression levels of these transcription factors.

These results confirm that *Lactobacillus kunkeei* NCHBL-003 culture fluid inhibits adipocyte differentiation by reducing the expression levels of these transcription factors.

### EXAMPLE 3: Preparation of Live Lactobacillus kunkeei NCHBL-003 and Lactobacillus plantarum NCHBL-004

To prepare live bacteria for oral administration to mice, *Lactobacillus kunkeei* NCHBL-003 and *Lactobacillus plantarum* NCHBL-004 (1×10⁸ CFU/ml) were cultured on solid plates (MRS agar) for 24 hours at 30°C. A single colony was then selected and pre-cultured in liquid medium (MRS broth). The pre-culture was conducted in 5 ml medium at 150 rpm and 30°C for 24 hours, after which it was diluted tenfold and main-cultured under the same conditions for 3 hours.

To adjust bacterial concentrations, a spectrophotometer was used to standardize the solution in PBS to an optical density (O.D.) of 0.6 at 600 nm. Under these conditions, *Lactobacillus kunkeei* NCHBL-003 contained approximately 1.57×10⁹ CFU/ml, and *Lactobacillus plantarum* NCHBL-004 contained approximately 1.48×10⁹ CFU/ml. Considering that each mouse would be orally administered 0.2 ml per day at concentrations of 1x10⁷ CFU/mouse and 1x10⁹ CFU/mouse per strain, the bacterial solution was centrifuged at 3,000 rpm for 15 minutes to prepare live bacterial suspensions.

### EXAMPLE 4: Experiment for Obesity Alleviation Effect (In Vivo)

### 4-1. Measurement of Weight Change in Obesity-Induced Mice

It is known that mice fed a high-fat diet (60% fat composition) gain more weight compared to mice on a normal diet. Consequently, obesity-induced mouse models via high-fat diet administration are widely used in obesity research.

Seven-week-old male C57BL/6 mice were fed either a normal diet or a high-fat diet along with administration of the live bacterial suspension. Two weeks after the start of the normal or high-fat diet, body weights were measured, and mice were allocated to groups to ensure equal average body weight per group. Starting from the third week of diet administration, body weights were measured weekly. Glucose tolerance tests were performed in week 10, insulin tolerance tests in week 15, and organ weights were measured at dissection in week 16.

The groups were set as follows, with each group containing 10 mice:

**TABLE 2**

| Group | Diet | Oral administration |
|---|---|---|
| G1 | General diet | PBS (phosphate buffered saline) |
| G2 | High-fat diet | PBS |
| G3 | High-fat diet | *Lactobacillus kunkeei* NCHBL-003 1*10⁷CFU/mouse |
| G4 | High-fat diet | *Lactobacillus kunkeei* NCHBL-003 1*10⁹CFU/mouse |
| G5 | High-fat diet | *Lactobacillus plantarum* NCHBL-004 1*10⁷CFU/mouse |
| G6 | High-fat diet | *Lactobacillus plantarum* NCHBL-004 1*10⁹CFU/mouse |

As a result of the instructions according to Table 2, it was observed that the group of high-fat diet (G2) had a significant weight gain compared to the group on a normal diet (G1).

**TABLE 3**

| | G1 | G2 | G3 | G4 | G5 | G6 |
|---|---|---|---|---|---|---|
| Weight (g, Week 16) | 34.25 | 44.25 | 42.64 | 39.21 | 40.4 | 40.73 |

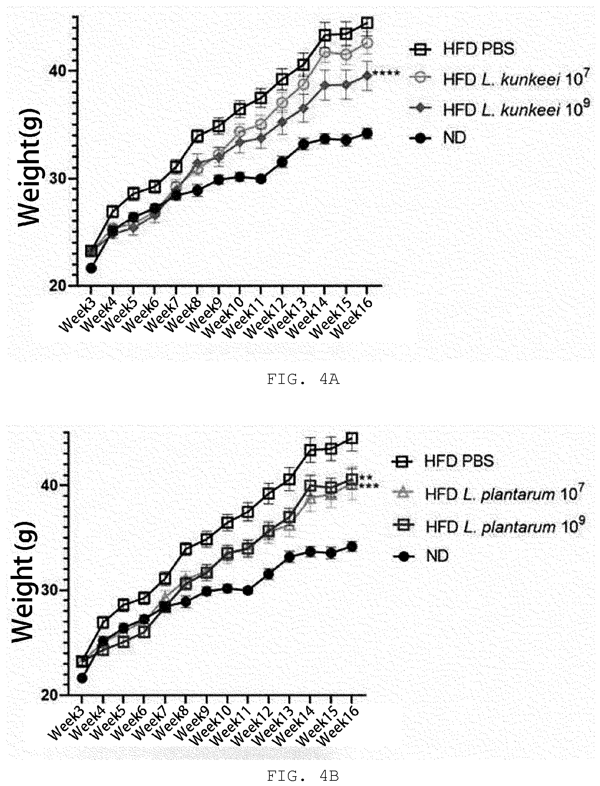

As shown in Table 3 and FIG. 4a, the group receiving a high-fat diet along with *Lactobacillus kunkeei* NCHBL-003 at a concentration of 1x10⁹ CFU (G4) exhibited a significant reduction in body weight compared to G2. The group administered a lower concentration of *Lactobacillus kunkeei* NCHBL-003 (1×10⁷ CFU, G3) showed an average weight reduction, but it was not statistically significant.

As shown in Table 2 and FIG. 4b, both groups fed *Lactobacillus plantarum* NCHBL-004 at concentrations of 1x10⁷ CFU and 1x10⁹ CFU (G5, G6) along with a high-fat diet showed significant weight reduction compared to G2.

These findings confirm that live *Lactobacillus kunkeei* NCHBL-003 and *Lactobacillus plantarum* NCHBL-004 bacteria induce a weight-reducing effect.

### 4-2. Evaluation for Blood Glucose Levels in Obesity-Induced Mice During Glucose Tolerance Test

When glucose is orally administered to mice, blood glucose levels temporarily rise but generally decrease due to normal insulin secretion. However, in high-fat diet-induced obese mouse models, blood glucose levels are known to remain elevated over time.

The mice in each group prepared in Example 4-1 were subjected to a glucose tolerance test after a 12-hour fasting period from 9 p.m. the previous day to 9 a.m. the next day. A 10% glucose solution (in PBS) sterilized via 0.2 µm filtration was prepared, and each mouse was orally administered 2 mg of glucose per gram of body weight (glucose/g, volume (µl) = body weight (g) × 20). The mice were returned to their cages, and blood samples were collected from the tail to measure blood glucose levels at 0, 15, 30, 60, 90, and 120 minutes post-administration.

In this study, the high-fat diet group (G2) was observed to retain significantly higher blood glucose levels compared to the normal diet group (G1).

**TABLE 4**

| Blood glucose level (mg/dL) in Glucose tolerance test | G1 | G2 | G3 | G4 | G5 | G6 |
|---|---|---|---|---|---|---|
| 0 min | 115.3 | 175 | 160.3 | 195.2 | 159.3 | 160.7 |
| 15 min | 263.5 | 400.3 | 356.2 | 372.2 | 383 | 378.7 |
| 30 min | 238.8 | 341.7 | 312.2 | 325.5 | 298.7 | 293.8 |
| 60 min | 188.8 | 260.7 | 234.8 | 223.2 | 207.8 | 207.2 |
| 90 min | 131.8 | 215.3 | 180.8 | 192.8 | 167.5 | 154.8 |
| 120 min | 98.5 | 187.7 | 160.3 | 168.5 | 140.5 | 152.5 |

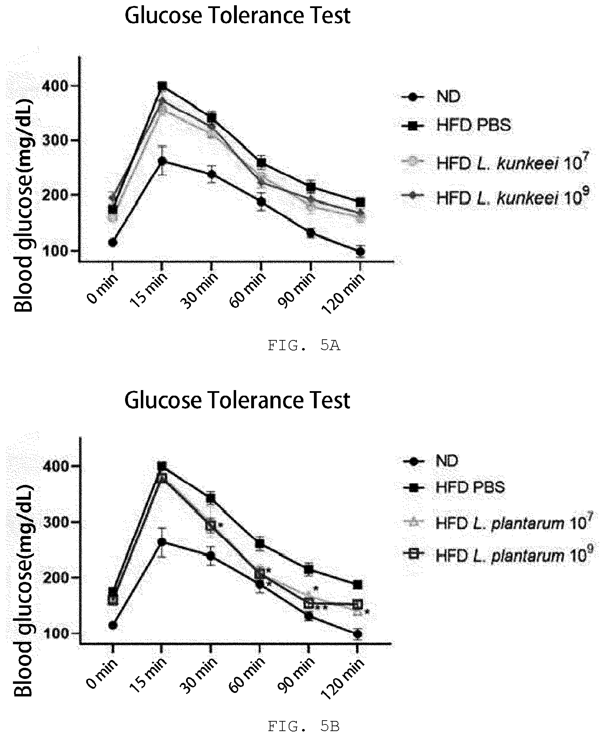

As shown in Table 4 and FIGS. 5a and 5b, the groups fed live *Lactobacillus kunkeei* NCHBL-003 and *Lactobacillus plantarum* NCHBL-004 bacteria alongside the high-fat diet (G3-6) showed reduced blood glucose levels compared to G2. In particular, *Lactobacillus plantarum* NCHBL-004 led to a significant reduction in blood glucose at each measured time point.

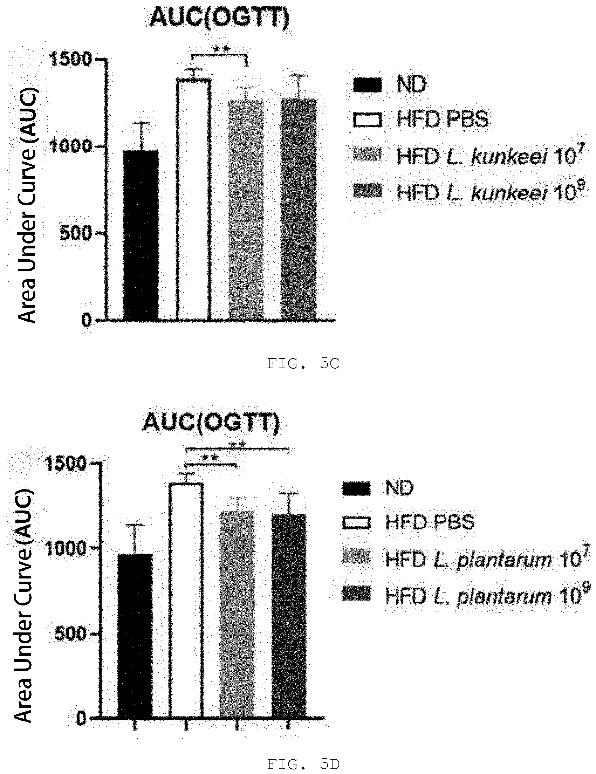

As indicated in FIGS. 5c and 5d, the area under the curve (AUC) for the glucose tolerance test demonstrated that the group receiving *Lactobacillus kunkeei* NCHBL-003 at 1x10⁷ CFU (G3) had a significant reduction in AUC compared to G2. Similarly, the groups administered *Lactobacillus plantarum* NCHBL-004 at 1x10⁷ and 1x10⁹ CFU (G3, G4) also showed significantly lower AUC values compared to G2.

These results from the glucose tolerance test confirm that both *Lactobacillus kunkeei* NCHBL-003 and *Lactobacillus plantarum* NCHBL-004 exhibit blood glucose-lowering effects in high-fat diet-induced obese mouse models.

### 4-3. Evaluation for Blood Glucose Levels in Obesity-Induced Mice During Insulin Tolerance Test

In high-fat diet-induced obese mouse models, blood glucose levels remain high. When insulin is administered intraperitoneally to such models, blood glucose temporarily decreases; however, in high-fat diet-induced obese models, this decrease is generally less pronounced.

Mice in each group from Example 4-1 were subjected to an insulin tolerance test after fasting for 4 hours, from 9 a.m. to 1 p.m. A sterilized insulin solution (in PBS) filtered through a 0.2 µm filter was prepared and administered intraperitoneally at a dose of 0.5 IU/kg (BW. Vol = 20 µl) using a 27-gauge sterile needle. Blood samples were collected from the tail, and blood glucose levels were measured at 0, 15, 30, 60, 90, 120, and 150 minutes post-administration.

In this study, the high-fat diet group (G2) was observed to exhibit significantly higher blood glucose levels compared to the normal diet group (G1).

**TABLE 5**

| Blood glucose level in Insulin tolerance test (mg/dL) | G1 | G2 | G3 | G4 | G5 | G6 |
|---|---|---|---|---|---|---|
| 0 min | 150.0 | 185.1 | 204.4 | 207.2 | 193.4 | 186.0 |
| 15 min | 104.0 | 211.3 | 226.1 | 186.9 | 171.8 | 173.7 |
| 30 min | 67.6 | 160.1 | 146.7 | 145.9 | 103.7 | 111.6 |
| 60 min | 65.9 | 147.3 | 129.3 | 120.0 | 94.9 | 100.7 |
| 90 min | 76.8 | 163.8 | 133.1 | 129.4 | 104.6 | 111.8 |
| 120 min | 83.7 | 160.9 | 133.5 | 143.0 | 115.4 | 126.3 |
| 150 min | 94.1 | 169.4 | 140.0 | 138.9 | 116.6 | 132.5 |

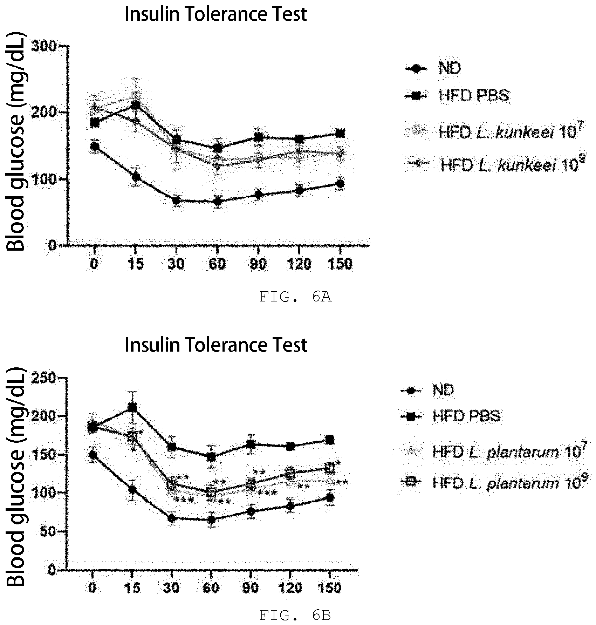

As shown in Table 5 and FIGS. 6a and 6b, the groups fed live *Lactobacillus kunkeei* NCHBL-003 and *Lactobacillus plantarum* NCHBL-004 bacteria along with the high-fat diet (G3-6) showed a reduction in blood glucose levels compared to G2. In particular, *Lactobacillus plantarum* NCHBL-004 demonstrated significant reductions in blood glucose at each time point measured.

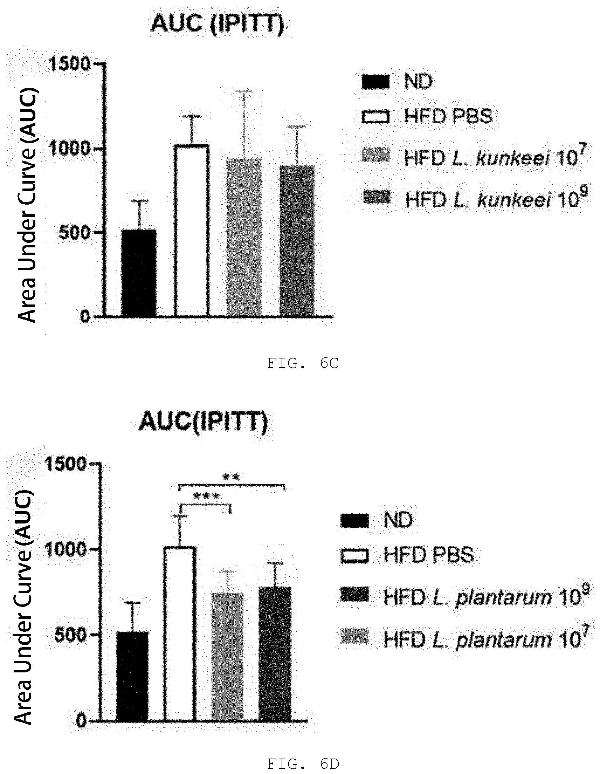

As shown in FIGS. 6c and 6d, the area under the curve (AUC) calculated from the insulin tolerance test indicated that while the groups receiving *Lactobacillus kunkeei* NCHBL-003 (G3, G4) had reduced blood glucose levels compared to G2, these reductions were not statistically significant. However, the groups administered *Lactobacillus plantarum* NCHBL-004 at concentrations of 1×10⁷ CFU and 1×10⁹ CFU (G5, G6) showed significantly lower AUC values compared to G2.

These results from the insulin tolerance test confirm that both *Lactobacillus kunkeei* NCHBL-003 and *Lactobacillus plantarum* NCHBL-004 have blood glucose-lowering effects in high-fat diet-induced obese mouse models.

### 4-4. Measurement of Organ Weights in Obesity-Induced Mice

Sixteen weeks after inducing obesity in mice via a high-fat diet, a necropsy was performed, and organ weights were measured for comparison.

For histological analysis, liver and adipose tissues were fixed in a 10% formalin solution, embedded in paraffin, and sectioned into 3 µm-thick slides. The sections were stained with hematoxylin and eosin (H&E) to stain the nuclei and cytoplasm, and images were captured at 100x magnification using an optical microscope with a mounted digital camera. The histopathological evaluation of non-alcoholic fatty liver disease (NAFLD) was based on the following criteria: steatosis (0-3), hepatocellular ballooning (0-3), and lobular inflammatory cell infiltration (0-2). The size of adipocytes in adipose tissue was measured using the ImageJ program.

Both liver and subcutaneous fat weights were higher in the high-fat diet group (G2) compared to the normal diet group (G1).

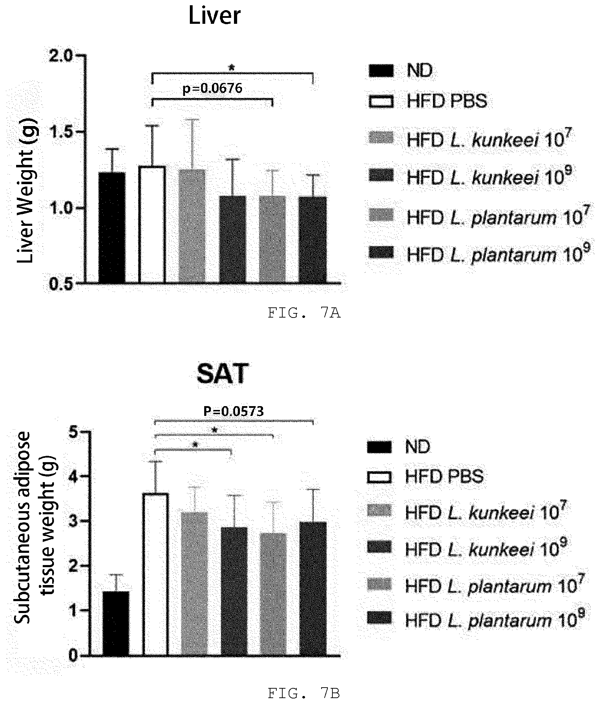

As shown in FIG. 7a, a reduction in liver weight was observed in the group receiving *Lactobacillus kunkeei* NCHBL-003 at a concentration of 1x10⁹ CFU (G4), though the reduction was not statistically significant. In the groups fed *Lactobacillus plantarum* NCHBL-004 (G5, G6) alongside the high-fat diet, liver weight reductions were observed, with a statistically significant reduction in the group administered 1x10⁹ CFU (G6).

As shown in FIG. 7b, a reduction in subcutaneous fat weight was observed in the groups receiving *Lactobacillus kunkeei* NCHBL-003 (G3, G4) with the high-fat diet, with a statistically significant reduction in the group given 1x10⁹ CFU (G4). Similarly, in the groups receiving *Lactobacillus plantarum* NCHBL-004 (G5, G6) with the high-fat diet, a reduction in subcutaneous fat weight was observed, with a statistically significant reduction in the group administered 1x10⁷ CFU (G5).

### 4-5. Evaluation of Clinical Symptoms of Fatty Liver in Obesity-Induced Mice

In high-fat diet-induced obese mouse models, lipid accumulation in liver tissue leads to clinical symptoms of non-alcoholic fatty liver disease (NAFLD).

Total RNA was extracted from adipose tissue using TRIzol reagent. The isolated RNA was quantified using a Nanodrop spectrophotometer, and cDNA synthesis from RNA was conducted with TOPscript RT DryMIX (Enzynomics, Korea) through a reverse transcription reaction. The synthesized cDNA was used as a template for amplification, with each primer and the TOPreal SYBR Green PCR kit (Enzynomics, Korea) added. Real-time PCR was performed using the Rotor-Gene Q (QIAGEN), and expression levels were analyzed with quantitative software.

The amplified products from real-time PCR were quantified using the comparative cycle threshold (Ct) method, with the housekeeping gene 18S rRNA serving as the internal control for normalization across samples. Primer sequences for each gene used in PCR are shown in Table 6.

**TABLE 6**

| SEQ ID NO: | Name | Sequence |
|---|---|---|
| 2 | 18S rRNA forward primer | AGGAGCTGAAGGGCCACGGG |
| 3 | 18S rRNA reverse primer | TGGGAACAGTGACGCGGGTC |

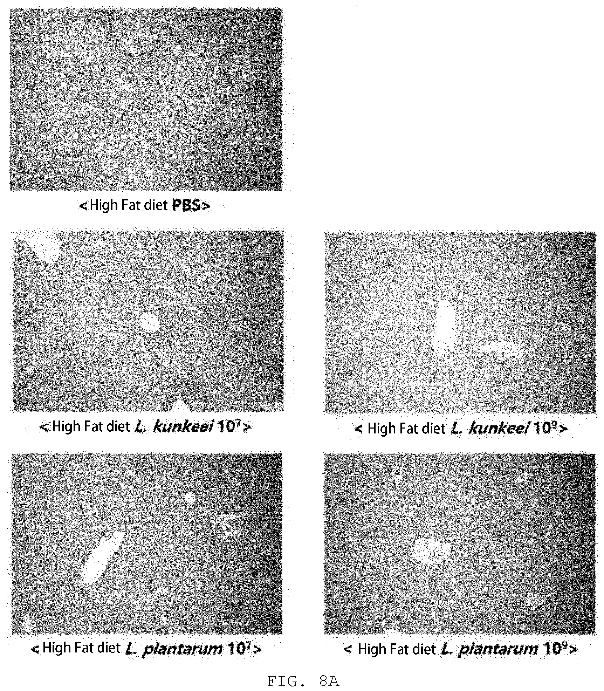

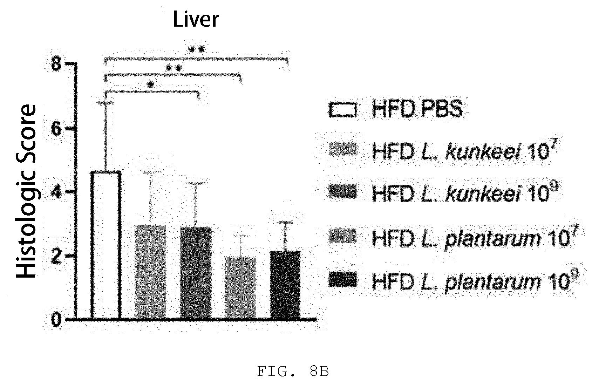

As shown in FIGS. 8a and 8b, administration of a high-fat diet for 16 weeks resulted in steatosis, hepatocellular ballooning, and lobular inflammatory cell infiltration in liver tissue. In groups fed with *Lactobacillus kunkeei* NCHBL-003 along with a high-fat diet, these clinical symptoms were reduced, with a statistically significant reduction observed in the group administered 1 × 10^9 CFU (G4). Similarly, in the groups fed *Lactobacillus plantarum* NCHBL-004 (G5, G6) along with a high-fat diet, clinical symptoms were reduced to a statistically significant degree compared to the high-fat diet-only group (G2).

### 4-6. Histological Evaluation of Adipose Tissue in Obesity-Induced Mice

In high-fat diet-induced obese mouse models, it is known that lipid accumulation in adipose tissue leads to an increase in adipocyte size.

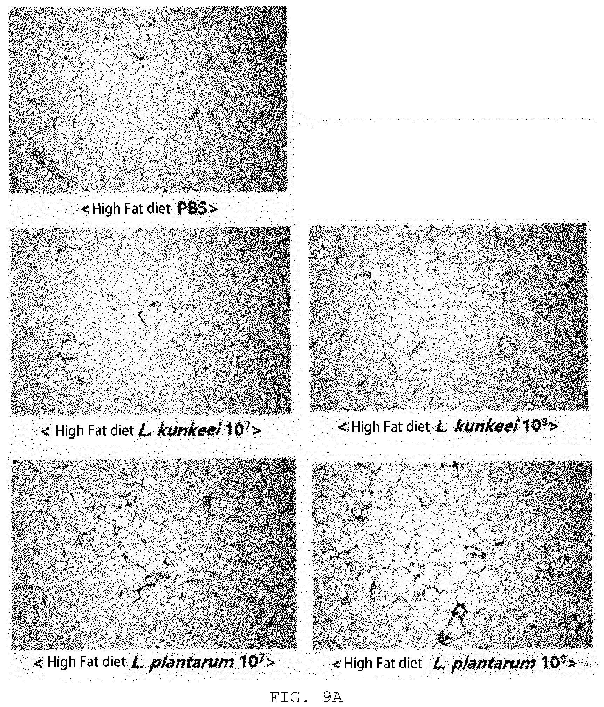

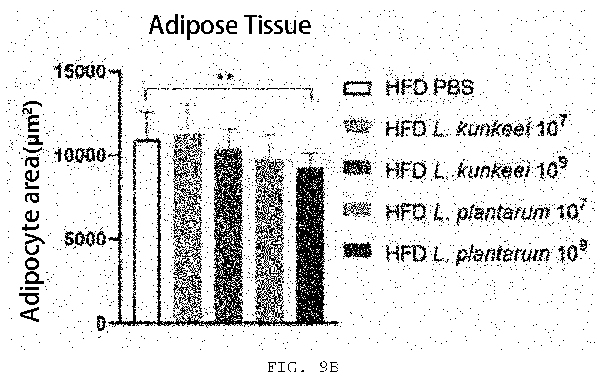

As shown in FIGS. 9a and 9b, the average size of adipocytes was reduced in the group administered *Lactobacillus kunkeei* NCHBL-003 at a concentration of 1x10⁹ CFU (G4) along with a high-fat diet. In addition, the groups fed *Lactobacillus plantarum* NCHBL-004 along with a high-fat diet showed a reduction in the average adipocyte size, with a statistically significant reduction observed in the group administered 1x10⁹ CFU (G6).

### 4-7. Evaluation for Gene Expression in Epididymal Adipose Tissue of Obesity-Induced Mice Treated with Lactobacillus kunkeei NCHBL-003

Sixteen weeks after initiating the experiment on the groups of mice prepared in Example 4-1, a necropsy was performed, and RNA was extracted from epididymal adipose tissue to assess the expression of metabolism-related genes. The primers used for expression analysis are shown in Table 7.

**TABLE 7**

| SEQ ID NO: | Name | Sequence |
|---|---|---|
| 4 | Adiponectin forward primer | AGGAGCTGAAGGGCCACGGG |
| 5 | Adiponectin reverse primer | TGGGAACAGTGACGCGGGTC |
| 6 | GLUT4 forward primer | ACATACCTGACAGGGCAAGG |
| 7 | GLUT4 reverse primer | CGCCCTTAGTTGGTCAGAAG |
| 8 | HSL forward primer | TGGGGAGCTCCAGTCGGAAGAGG |
| 9 | HSL reverse primer | CATTAGACAGCCGCCGTGCTG |
| 10 | ATGL forward primer | CTCATTCGCTGGCTGCGGCT |
| 11 | ATGL reverse primer | CCCCAGTGACCAGCGCTGTG |
| 12 | CPT1a forward primer | ACCACTGGCCGCATGTCAAG |
| 13 | CPT1a reverse primer | AGCGAGTAGCGCATAGTCAT |

It is well known that adiponectin is primarily produced in adipocytes, playing a crucial role in regulating glucose and lipid metabolism, enhancing insulin sensitivity, and decreasing in cases of metabolic syndrome. GLUT4 (glucose transporter type 4) facilitates glucose uptake into peripheral tissues in response to insulin. In cases of insulin resistance, the translocation of GLUT4 from the cytoplasm to the cell membrane is inhibited.

Hormone-sensitive lipase (HSL) and adipose triglyceride lipase (ATGL) are essential enzymes in lipid metabolism, primarily active in adipose tissue, where they contribute to triglyceride breakdown. Carnitine palmitoyltransferase 1A (CPT1A) promotes mitochondrial fatty acid β-oxidation.

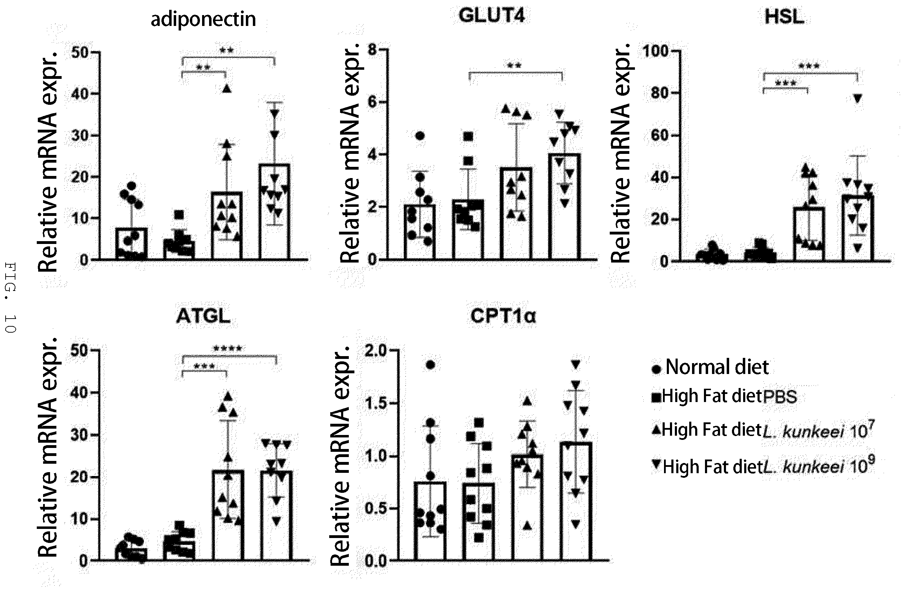

As shown in FIG. 10, administration of *Lactobacillus kunkeei* NCHBL-003 along with a high-fat diet resulted in a statistically significant increase in the expression of these genes in epididymal adipose tissue. However, the increase in GLUT4 expression in the group administered 1×10⁷ CFU (G3) and the increase in CPT1A expression in both G3 and G4 were not statistically significant. These findings confirm that *Lactobacillus kunkeei* NCHBL-003 regulates the expression of metabolism-related genes in adipose tissue.

### 4-8. Evaluation for Gene Expression in Epididymal Adipose Tissue of Obesity-Induced Mice Treated with Lactobacillus plantarum NCHBL-004

Sixteen weeks after inducing obesity in mice via a high-fat diet, necropsy was performed, and RNA was extracted from epididymal adipose tissue to assess the expression levels of genes related to adipocyte differentiation and inflammation.

**TABLE 8**

| SEQ ID NO: | Name | Sequence |
|---|---|---|
| 14 | C/EBPα forward primer | GAACAGCAACGAGTACCGGGTA |
| 15 | C/EBPα reverse primer | CCATGGCCTTGACCAAGGAG |
| 16 | PPARγ forward primer | GGTGAAACTCTGGGAGATTC |
| 17 | PPARγ reverse primer | CAACCATTGGGTCAGCTCTT |
| 18 | aP2 forward primer | ACATGATCATCAGCGTAAATGGG |
| 19 | aP2 reverse primer | TCATAACACATTCCACCACCAGC |
| 20 | TNF-α forward primer | CGTCAGCCGATTTGCTATCT |
| 21 | TNF-α reverse primer | CGGACTCCGCAAAGTCTAAG |
| 22 | Caspase1 forward primer | AGGAATTCTGGAGCTTCAATCAG |
| 23 | Caspase1 reverse primer | TGGAAATGTGCCATCTTCTTT |

Transcription factors PPARγ (peroxisome proliferator-activated receptor-γ) and C/EBPα (CCAAT-enhancer binding protein), which are involved in the differentiation of adipocytes, play key roles in promoting adipocyte differentiation by regulating the expression of target genes. aP2 (adipocyte protein 2) is a gene associated with mature adipocytes.

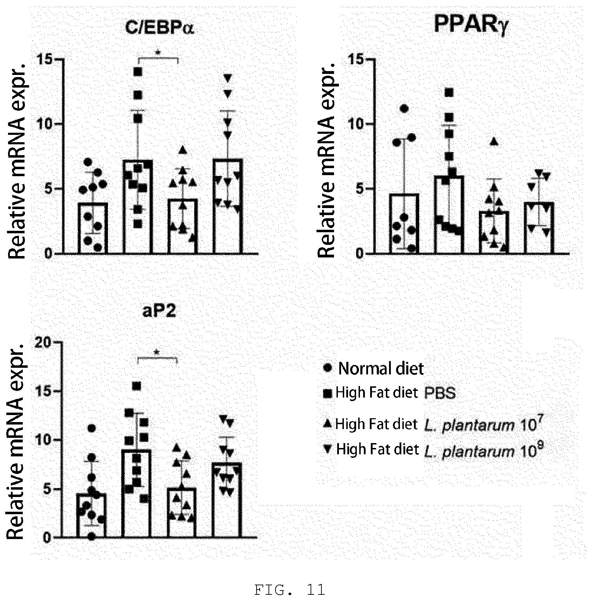

As shown in FIG. 11, administration of *Lactobacillus plantarum* NCHBL-004 at a concentration of 1×10⁷ CFU (G5), along with the high-fat diet, resulted in a reduction in the expression of these genes, with statistically significant reductions observed in C/EBPα and aP2. When *Lactobacillus plantarum* NCHBL-004 was administered at 1x10⁹ CFU (G6), reductions in PPARγ and aP2 gene expression were observed, although these were not statistically significant.

In the context of metabolic diseases, inflammation is often present at chronic, low levels, promoting insulin resistance and contributing to the pathophysiology of obesity-related metabolic disorders. TNFα (tumor necrosis factor α) and MCP1 (monocyte chemoattractant protein-1) are pro-inflammatory cytokines mainly secreted by adipocytes, while caspase-1 plays a crucial role in macrophage-induced inflammation within adipose tissue.

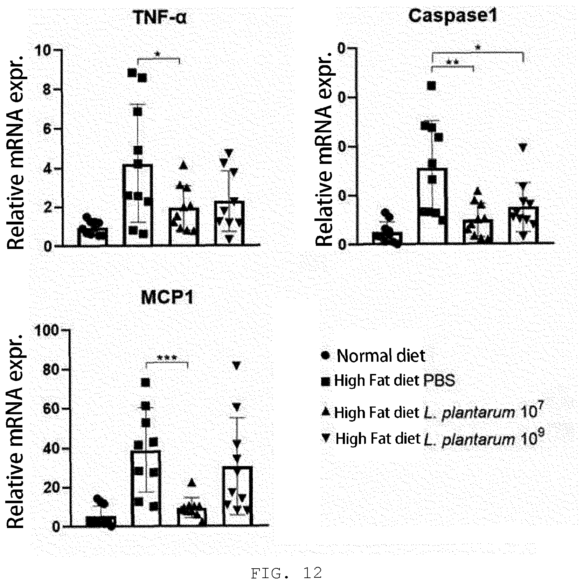

As shown in FIG. 12, administration of *Lactobacillus plantarum* NCHBL-004 at 1x10⁷ CFU (G5) significantly reduced the expression of these inflammation-related genes. When live *Lactobacillus plantarum* NCHBL-004 was added administered at 1x10⁹ CFU (G6), the expression of these genes decreased, with a statistically significant reduction observed particularly in caspase-1 expression.

These findings confirm that administration of *Lactobacillus plantarum* NCHBL-004 regulates the expression of genes related to adipocyte differentiation and inflammation in adipose tissue.

### Industrial Applicability

The present disclosure relates to a composition including *Lactobacillus plantarum* NCHBL-004 strain or a culture thereof for the prevention, treatment, or alleviation of metabolic diseases. More specifically, the present disclosure relates to a technology for applying either a culture or live bacteria of *Lactobacillus plantarum* NCHBL-004 to the prevention, treatment, or alleviation of metabolic diseases, including obesity, diabetes, and non-alcoholic fatty liver disease (NAFLD).

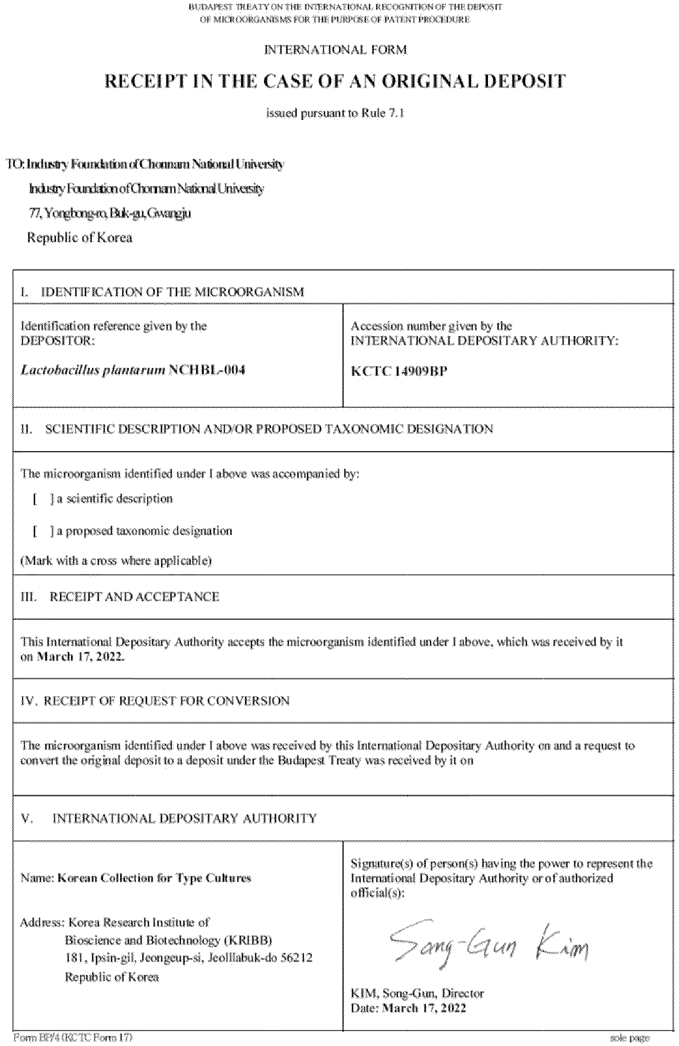
BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

RECEIPT IN THE CASE OF AN ORIGINAL DEPOSIT

issued pursuant to Rule 7.1

TO: Industry Foundation of Chonnam National University
Industry Foundation of Chonnam National University
77, Yongbong-ro, Buk-gu, Gwangju
Republic of Korea

I. IDENTIFICATION OF THE MICROORGANISM
Identification reference given by the
DEPOSITOR:

Lactobacillus plantarum NCHBL-004 | Accession number given by the
INTERNATIONAL DEPOSITARY AUTHORITY:

KCTC 14909BP
II. SCIENTIFIC DESCRIPTION AND/OR PROPOSED TAXONOMIC DESIGNATION
The microorganism identified under I above was accompanied by:
[ ] a scientific description
[ ] a proposed taxonomic designation
(Mark with a cross where applicable)
III. RECEIPT AND ACCEPTANCE
This International Depositary Authority accepts the microorganism identified under I above, which was received by it
on March 17, 2022.
IV. RECEIPT OF REQUEST FOR CONVERSION
The microorganism identified under I above was received by this International Depositary Authority on and a request to
convert the original deposit to a deposit under the Budapest Treaty was received by it on
V. INTERNATIONAL DEPOSITARY AUTHORITY
Name: Korean Collection for Type Cultures

Address: Korea Research Institute of
Bioscience and Biotechnology (KRIBB)
181, Ipsin-gil, Jeongeup-si, Jeollabuk-do 56212
Republic of Korea | Signature(s) of person(s) having the power to represent the
International Depositary Authority or of authorized
official(s):


KIM, Song-Gun, Director
Date: March 17, 2022

Form BP/4 (KCTC Form 17) sole page

## Claims

1. A pharmaceutical composition for preventing or treating metabolic diseases, the composition comprising *Lactobacillus plantarum* NCHBL-004 strain or a culture thereof.

2. The pharmaceutical composition of claim 1, wherein the strain is deposited under accession number KCTC14909BP.

3. The pharmaceutical composition of claim 1, wherein the strain is in a form of live bacteria, dead bacteria, or a mixture thereof.

4. The pharmaceutical composition of claim 1, wherein the composition comprises the strain at a concentration of 5×10⁵ to 5×10¹¹ CFU/ml.

5. The pharmaceutical composition of claim 1, wherein the metabolic disease is at least one selected from the group consisting of obesity, diabetes, non-alcoholic fatty liver disease (NAFLD), non-alcoholic steatohepatitis (NASH), hypertension, hyperlipidemia, cardiovascular disease, and hyperinsulinemia.

6. A health-functional food composition for alleviating metabolic diseases, the composition comprising *Lactobacillus plantarum* NCHBL-004 strain or a culture thereof.

7. The health-functional food composition of claim 6, wherein the strain is deposited under accession number KCTC14909BP.

8. The health-functional food composition of Claim 6, wherein the strain is in a form of live bacteria, dead bacteria, or a mixture thereof.

9. The health-functional food composition of Claim 6, wherein the composition comprises the strain at a concentration of 5x10⁵ to 5x10¹¹ CFU/ml.

10. The health-functional food composition of claim 6, wherein the metabolic disease is at least one from the group consisting of obesity, diabetes, non-alcoholic fatty liver disease (NAFLD), non-alcoholic steatohepatitis (NASH), hypertension, hyperlipidemia, cardiovascular disease, and hyperinsulinemia.
